# FASCICULE DE BREVET EUROPEEN

(11) **EP 0 932 423 B1**
(45) Date de publication et mention de la délivrance du brevet: **03.12.2003**
(21) Numéro de dépôt: 97937396.6
(22) Date de dépôt: 08.09.1997
(51) Int. Cl.: A61M 5/142

(54) **POMPE PERISTALTIQUE MINIATURE A USAGE MEDICAL**
MINIATURISIERTE PERISTALTIKPUMPE ZUR MEDIZINISCHEN ANWENDUNG
MINIATURE PERISTALTIC PUMP FOR MEDICAL USE

(30) Priorité: 10.09.1996 FR 9611172
(43) Date de publication de la demande: 04.08.1999
(73) Titulaire: Precimedix S.A., 2000 Neuchâtel (CH)
(72) Inventeur: RAY, Claude, . (CH); TAILLARD, Christian, F-25500 Les Fins (FR)
(74) Mandataire: Gresset, Jean
(86) Numéro de dépôt international: CH9700328
(87) Numéro de publication internationale: WO98010807

(56) Documents cités:
- EP-A- 0 521 184
- WO-A-94/06491
- DE-A- 3 022 498
- US-A- 4 722 734
- US-A- 5 083 908

## Description

La présente invention se rapporte aux pompes à usage médical. Elle concerne, plus particulièrement, une pompe péristaltique miniature pour l'injection de solutions médicamenteuses.

Les pompes miniatures ou micropompes à usage médical sont connues depuis plusieures années. Légères et de faibles dimensions, elles sont portées par le patient de façon discrète et sans inconfort et permettent de lui administrer, par voie sous-cutanée ou intraveineuse, en continu ou selon un programme déterminé, des quantités contrôlées de solutions médicamenteuses, sans qu'il soit pour autant alité et relié à un appareil encombrant, coûteux et bruyant.

De telles pompes sont, le plus souvent, de type péristaltique rotatif dont le principe consiste à disposer d'un tuyau en matière plastique déformable relié à un réservoir contenant la solution et à l'écraser localement contre une pièce d'appui de forme arrondie au moyen de galets presseurs montés sur un rotor entraîné par un moteur agissant par l'intermédiaire d'un train d'engrenages. Le liquide est ainsi aspiré du réservoir et refoulé vers la sortie pour être injecté dans le patient.

Les pompes dites à cassette sont les plus courantes pour cette application. Elles comportent deux parties, d'une part, la pompe proprement dite, avec le moteur, l'électronique de pilotage, la pile et la tête de pompe formée du rotor et des galets presseurs et, d'autre part, la cassette qui s'enclipse sur la pompe et comprend le tuyau et la pièce d'appui. Le réservoir est soit intégré dans la cassette lorsqu'il est de petites dimensions, soit disposé librement à l'extérieur lorsqu'il est de grandes dimensions.

Les brevets EP 447 909, EP 521 184 et WO 94/06491 décrivent des pompes miniatures de ce type.

Dans les trois cas, la cassette comporte, assemblés de manière définitive, le tuyau, la pièce d'appui et le réservoir. Les pompes des deux brevets EP ne sont utilisables qu'une seule fois car, une fois accouplés, les deux modules ne peuvent plus être désaccouplés. A la fin du traitement ou lorsque la cassette est vide, on jette le tout, y compris donc le moteur, la tête de pompe, la pièce d'appui, le train d'engrenages et le circuit qui pourtant sont tous capables de fonctionner à nouveau. Quant à la pompe du brevet WO, sa cassette est jetable en même temps que son réservoir. Ainsi, dans ces trois pompes, on jette des composants qui pourraient encore être utilisés tels quels puisqu'ils ne sont pas usés et n'ont jamais été en contact avec le médicament injecté dans le malade.

Les documents US 4 817 057 et EP 120 284 décrivent, par ailleurs, des pompes péristaltiques à cassette de grandes dimensions, c'est à dire non portables. Bien que le tuyau puisse être aisément inséré dans - ou enlevé de - la cassette, il est muni d'un embout de raccordement à la tubulure provenant du réservoir et sa portion soumise à l'action péristaltique est spécialement conçue pour résister à une exposition prolongée à l'effet de compression des galets. Une telle construction présente des inconvénients car, non seulement, elle n'interdit pas la réutilisation du tuyau pour administrer des solutions médicamenteuses différentes à des patients différents, mais encore, les opérations d'interconnexion des deux tubes accroissent le risque de pollution du circuit par des bactéries.

Lors de la conception d'une pompe péristaltique miniature, il est particulièrement important de veiller à l'optimisation du couplage entre les galets et le tuyau qu'ils viennent écraser contre la pièce d'appui.

Des mesures ont montré que la pression minimale sur un galet de 5mm de diamètre nécessaire pour faire écouler un liquide dans un tuyau plastique ayant un diamètre interne de 1.47 mm et un diamètre externe de 1.96 mm est de 95 gr. La force de traction correspondante exercée par le galet est de 15 gr. La pression sur le galet peut monter jusqu'à 150 gr sans faire augmenter proportionnellement la force de traction qui passe alors seulement de 15 à 20 gr. Mais au delà de cette limite, la force de traction augmente très rapidement. Elle passe, en effet, à 50 gr pour une pression de 200 gr, après quoi les mesures deviennent impossibles.

De telles constatations s'expliquent aisément par le fait qu'une fois le tuyau comprimé jusqu'à sa complète obturation, toute augmentation de pression provoque la déformation du matériau plastique et la force de traction correspondante augmente alors en relation avec son module d'élasticité.

Ainsi, toute variation de position du galet par rapport au tuyau au delà de celle qui réalise sa complète obturation sollicite fortement le rotor porte-galets et son moteur, ce qui provoque rapidement son bloquage et donc l'arrêt de la pompe. L'effet est d'autant plus marqué dans les pompes miniatures qui sont équipées de moteurs évidemment moins puissants que ceux des pompes non portables.

A l'inverse, toute variation de position du galet en deçà de celle qui réalise la complète obturation du tuyau ne permet pas un écoulement normal du liquide.

Il est donc très important, pour obtenir une pompe à fonctionnement sûr, que la distance séparant le galet de la pièce d'appui soit parfaitement définie et constante afin d'éviter le bloquage pur et simple ou un débit insuffisant.

Si donc la piéce d'appui et le galet sont fixes, des tolérances de fabrication extrèmement sévères sont nécessaires et augmentent ainsi très sensiblement le prix de revient de la pompe.

Il est donc préférable d'accepter des tolérances plus légères et de prévoir des moyens permettant de compenser automatiquement tout écart de position entre la pièce d'appui et le galet.

Les brevets EP 388 787 et EP 447 909 (déjà cité) décrivent succintement des arrangements pour résoudre ce problème.

Le brevet EP 388 787 montre une pièce d'appui présentant la forme d'un crochet articulé à l'une de ses extrémités par une goupille et pressé contre le tuyau à l'aide d'un ressort comprimé par une vis. Cette pièce d'appui étant pratiquement toujours sollicitée par deux galets, elle ne peut assurer la compensation des écarts de position pour chaque galet séparément.

Le brevet EP 447 909 montre que les galets sont montés sur leur axe avec un léger jeu radial leur permettant un certain débattement radial et que des ressorts à lame individuels, agissant directement sur leur partie centrale, les sollicitent vers l'extérieur. Une telle disposition présente le double inconvénient de compliquer le montage du rotor et de l'amener à glisser sur le tuyau plutôt que de tourner. Il est aussi indiqué, dans ce document, que les ressorts peuvent être remplacés par une pièce élastique unique, dont aucune description n'est cependant fournie et qu'ils peuvent être carrément omis car les galets sont alors déplacés radialement par l'élasticité inhérente du tuyau lui-même. Ceci montre que l'importance du problème n'a pas été pleinement perçue.

Un autre problème qui se pose lors de la conception d'une pompe péristaltique miniature est celui de sa purge, c'est à dire de l'évacuation de l'air contenu encore dans sa tubulure, avant l'introduction de l'aiguille dans le patient. La vitesse de rotation du rotor étant très faible, généralement inférieure à 1 tour/minute, cette opération doit, pour ne pas prendre trop de temps, être effectuée en faisant tourner rapidement le rotor par des moyens externes. Dans le brevet EP 388 787, il est prévu une roue qui est solidaire du rotor et comporte une série de trous dans lesquels on peut introduire la pointe d'un stylo, par exemple, pour faire tourner la roue rapidement. La solution est, certes, intéressante, mais aucune disposition n'est prévue pour éviter que cette opération n'endommage le train d'engrenages.

La présente invention a pour but de fournir une pompe péristaltique portable à usage médical qui est exempte des inconvénients des pompes de d'art antérieur.

La pompe selon l'invention comporte un premier et un deuxième module dotés chacun de moyens permettant leur accouplement, le premier module, dit module pompe, comprenant un rotor équipé d'au moins un galet presseur, des moyens d'entraînement dudit rotor et des moyens de commande desdits moyens d'entraînement, le deuxième module, dit module cassette, comprenant une pièce d'appui munie d'une portion arrondie qui, une fois les deux modules accouplés, est sensiblement concentrique audit rotor.

Cette pompe est principalement caractérisée par le fait que :
- le module cassette comporte deux canaux ménagés le long de ses côtés pour recevoir un tuyau flexible relié à - et faisant partie indissociable d' - un réservoir de solution médicamenteuse et le positionner automatiquement de manière à ce qu'au moment de l'accouplement des deux modules, il s'applique contre ladite portion arrondie pour y être comprimé par ledit galet, et
- lesdits lesdits canaux comportent des portions coudées profilées et dimensionnées pour que des portions de forme correspondante dudit tuyau y prennent place et y soient retenues par enclipsage,
- le tuyau et le réservoir formant ainsi un troisième module de ladite pompe péristaltique, dit module réservoir, qui peut être aisément échangé avec un autre après usage.

Ainsi, la pompe selon l'invention présente l'avantage, par rapport aux pompes miniatures de l'art antérieur mentionnées ci-dessus, de permettre la réutilisation du module cassette, seul étant jeté l'ensemble formé par le tuyau et le réservoir. Par rapport aux pompes non portables de l'art antérieur, elle présente l'avantage d'éliminer complètement les risques de réutilisation du tuyau et de contamination.

D'autres caractéristiques de la présente invention ressortiront de la description qui va suivre, faite en regard des dessins annexés et donnant, à titre explicatif mais nullement limitatif, une forme préférée de réalisation de cette pompe. Sur ces dessins:
- la figure 1 est vue générale extérieure du module pompe;
- la figure 2 représente le module réservoir;
- la figure 3 représente le module cassette;
- la figure 4 montre la façon dont les trois modules sont accouplés;
- la figure 5 est un détail en coupe de la figure 4 lorsque l'accouplement est réalisé;
- la figure 6 est une vue générale de la pompe assemblée;
- la figure 7 est une vue de dessus du module pompe;
- la figure 8 est une vue en coupe du module pompe selon la ligne VIII-VIII de la figure 7;
- la figure 9 est une vue de dessus du rotor du module pompe;
- la figure 10 montre le module électronique de commande de la pompe;
- la figure 11 est un schéma du circuit de commande de la pompe;
- enfin, la figure 12 illustre une variante de réalisation du module réservoir.

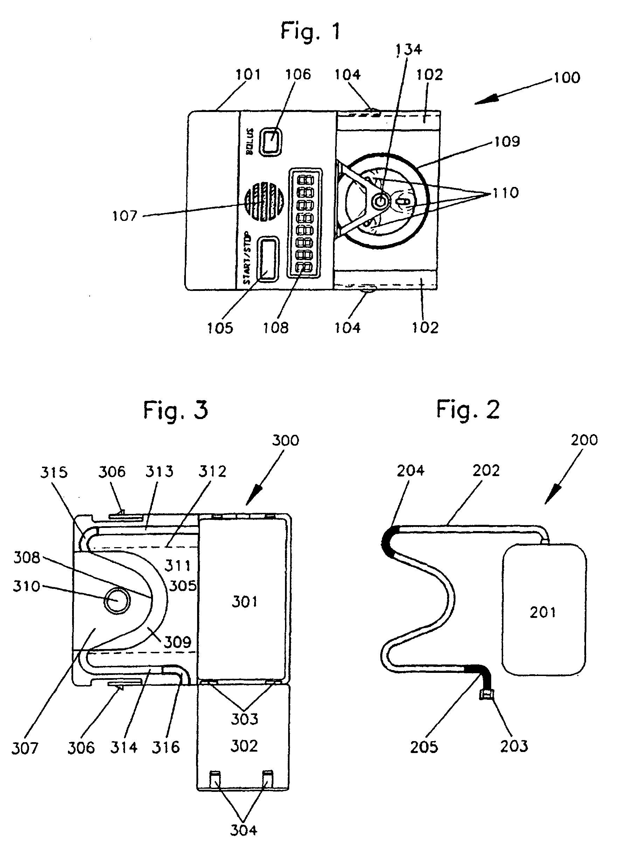

Ainsi que le montrent les figures 1 à 5, la pompe miniature selon l'invention est constituée de trois modules, un module pompe 100, un module réservoir 200 et un module cassette 300 qui seront aussi appelés respectivement pompe, réservoir et cassette. Le réservoir 200 se place de façon amovible dans la cassette 300 qui, à son tour, s'accouple de façon amovible à la pompe 100.

Pour fixer les idées et à simple titre d'exemple, la pompe miniature assemblée a une longueur de 110 mm, une largeur de 55 mm et une épaisseur de 13 mm pour une capacité de réservoir de 10 ml.

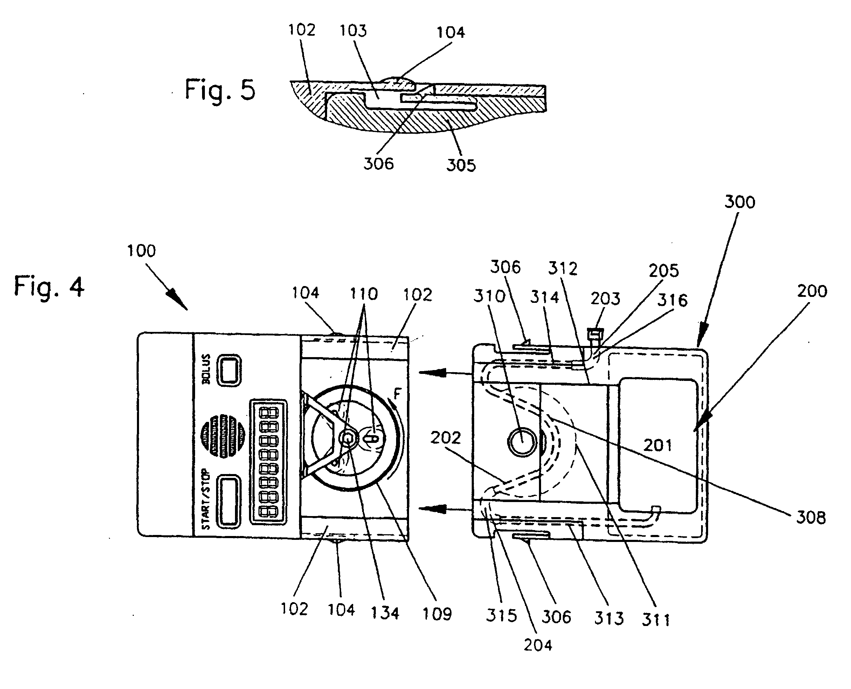

Le module pompe 100, représenté sur la figure 1, comporte un boîtier en plastique rigide 101 dont le fond se prolonge d'un côté pour former la base de deux coulisses parallèles 102 servant à la mise en place du module cassette 300 à la manière d'un tiroir. Ainsi qu'il apparaît mieux sur la figure 5, chaque coulisse 102 est percée d'une ouverture 103 dans laquelle est ménagée une languette souple 104 formant un bouton poussoir destiné au dégagement de la cassette lorsqu'elle doit être désaccouplée de la pompe.

Sur sa face supérieure, le boîtier 101 comporte un bouton "START/STOP" 105 servant à commander le démarrage et l'arrêt de la pompe, un bouton "BOLUS" 106 servant à déclencher l'administration de doses additionnelles de solution, un avertisseur acoustique 107 et un affichage LCD 108.

Le boitier 101 laisse apparaître, entre ses deux coulisses 102, un rotor 109 portant trois galets 110 et formant la tête de la pompe. Une description détaillée en sera donnée plus loin en même temps que celle de ses moyens d'entraînement.

Le module réservoir 200, représenté sur la figure 2, est formé d'une poche en plastique 201 ayant, dans le mode de réalisation représenté, un volume de 10 ml et d'un tuyau en plastique souple 202 dont une extrémité est reliée à la poche et dont l'autre, destinée à être connectée à une aiguille d'injection sous-cutanée ou intraveineuse, est obturée par un bouchon 203. Le tuyau comporte, en outre, en surépaisseur, deux raccords moulés rigides en forme de coude 204 et 205 servant à son enclipsage dans la cassette 300, ainsi qu'il apparaîtra mieux plus tard.

Le module cassette 300, représenté vu de dessous sur la figure 3, est réalisé en plastique rigide et comporte un logement 301 destiné à recevoir la poche plastique 201. Il a sensiblement la même épaisseur et la même largeur que le boîtier 101 et possède, côté fond, un couvercle 302 monté sur deux charnières 303 et muni, à l'autre extrémité, de languettes 304 assurant sa fermeture par enclipsage. Le logement 301 se prolonge par un plateau 305 profilé et dimensionné pour prendre place, comme un tiroir, entre les coulisses 102 du boîtier. Deux languettes 306, ménagées sur ses côtés, y permettent son enclipsage par arrimage de leur extrémité dans les ouvertures 103, ainsi que le montre également la figure 5.

Le plateau 305 est percé, côté fond, d'un logement 307, en forme générale de U, dimensionné de manière à recevoir le rotor 109 du module pompe. II comporte, à cet effet, une partie centrale arrondie 308 de 120 degrés environ et dont le rayon est légèrement supérieur à celui du cercle que parcourt la face externe des galets 110. C'est sur cette portion arrondie 308 qu'en fonctionnement, les trois galets viennent tour à tour écraser le tuyau flexible 202. Un dégagement supplémentaire arrondi 309 est prévu autour de la portion 308 pour recevoir la partie inférieure du rotor 109, de plus grand diamètre que le cercle parcouru par les galets, comme il apparaîtra plus loin.

Le fond du logement 307, qui forme la paroi supérieure du module, est percé, au centre de la portion arrondie 308, d'un orifice circulaire 310 au regard de l'endroit où prend place l'axe du rotor 109 afin de permettre la purge de la pompe avant usage, comme il sera décrit plus loin. Cet orifice est obturable par un couvercle 311 coulissant dans un évidement 312 de la paroi supérieure du module.

Le plateau 305 comporte également deux canaux 313 et 314 ménagés chacun dans le fond d'un de ses côtés parallèlement à celui-ci. Le canal 313 relie le logement 301 à la partie antérieure du logement 307. Avant de déboucher dans celle-ci, il présente un portion coudée 315, de plus grand diamêtre, formée et dimensionnée de manière à recevoir et maintenir par enclipsage le raccord rigide 204 du tuyau souple 202. Le canal 314 part de la partie antérieure du logement 307 à l'opposé de l'endroit où arrive l'autre canal 313 et débouche à l'extérieur après une portion coudée 316, de plus grand diamêtre, formée et dimensionnée de manière à recevoir et maintenir par enclipsage le raccord rigide 205 du tuyau 202.

On se référera maintenant plus spécialement aux figures 4, 5 et 6 qui montrent la façon dont les trois modules précédemment décrits s'accouplent pour former la pompe miniature selon l'invention.

Le module réservoir 200 est, tout d'abord, mis en place dans le module cassette 300. La poche plastique 201 est introduite dans son logement 301 et le tuyau souple 202 positionné dans les canaux 313 et 314 en veillant à ce que ses deux raccords rigides 204 et 205 prennent place et s'enclipsent respectivement dans les portions coudées 315 et 316 du plateau 305. Après avoir refermé le couvercle 302, il ne reste plus alors qu'à insérer la cassette dans les coulisses 102 du module pompe 100 jusqu'à ce que les languettes 306 s'accrochent dans les ouvertures 103 (figure 5). L'extrémité de la partie inférieure du rotor 109 vient occuper le dégagement 309 du plateau 305, tandis que la portion centrale du tuyau souple 202 se trouve automatiquement emprisonnée entre le rotor 109 et la pièce d'appui 308. Lorsque le rotor sera mis en rotation dans le sens de la flèche F (sens inverse des aiguilles d'une montre), les galets 110 viendront tour à tour comprimer le tuyau 102 pour pousser vers l'extérieur la solution qu'il contient.

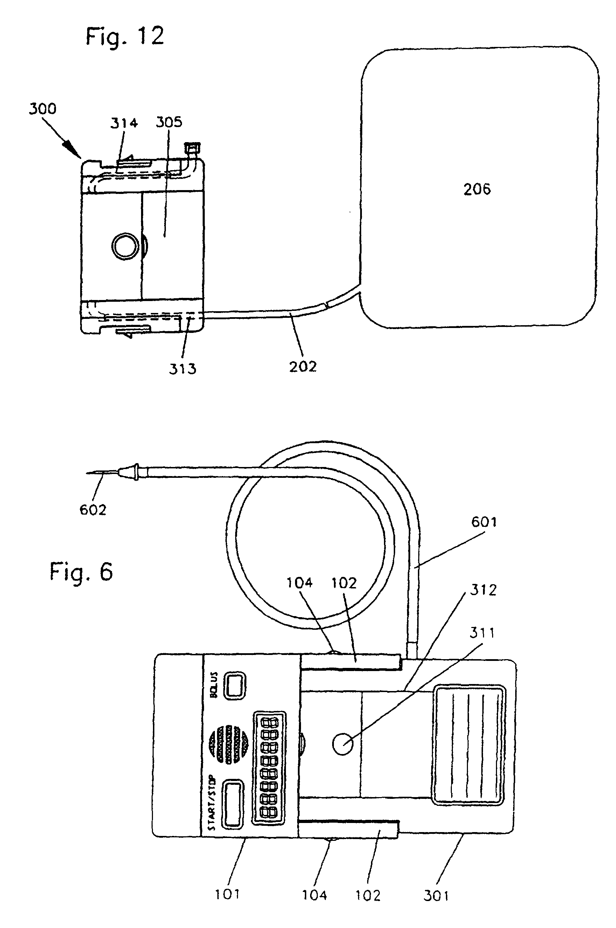

La pompe étant ainsi chargée, le bouchon 203 du tuyau est enlevé et remplacé, comme le montre la figure 6, par un tuyau 601 se terminant par une aiguille 602. Avant d'introduire cette dernière dans le patient, il est impératif d'évacuer l'air présent dans le circuit et d'y faire arriver la solution à injecter. La vitesse de rotation normale du rotor 109 étant très faible (0.625 tour/minute), ainsi qu'il sera décrit plus loin, cette opération de purge doit, pour ne pas prendre trop de temps, être effectuée en faisant tourner rapidement le rotor par des moyens externes. L'extrémité supérieure de l'axe de ce dernier présente, à cet effet, une ouverture de forme 134 apparaissant dans l'ouverture 310 de la cassette et qui permet, à l'aide d'un outil approprié, la rotation rapide du rotor jusqu'à ce que des gouttes de la solution à injecter sortent de l'aiguille 602. Celle-ci peut être alors introduite dans le patient, après quoi la pompe est mise en marche en appuyant sur le bouton 105 qui commande la mise en rotation du rotor 109 par des moyens qui seront décrits plus loin en détail.

En fin de traitement, le désaccouplement des modules pompe 100 et cassette 300 est réalisé en appuyant sur les deux boutons 104 de manière à dégager les languettes 306 des ouvertures 103. La cassette peut alors être extraite en la glissant vers l'extérieur avant d'en retirer le réservoir 200 qui peut être remplacé par un autre, la cassette et la pompe pouvant être réutilisées pour un autre traitement.

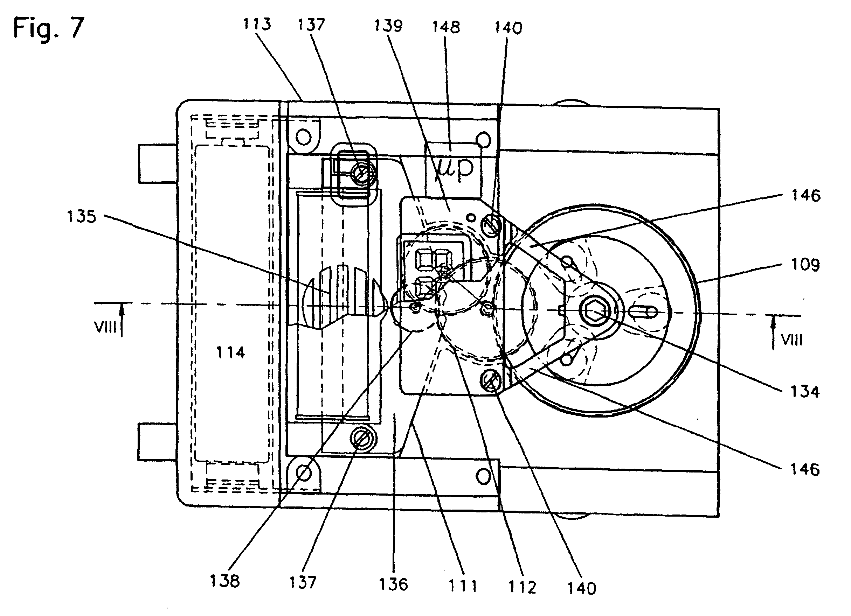

Le module pompe 100 va être maintenant décrit en se référant aux figures 7 à 11 qui montrent le rotor 109, un moteur pas à pas 111, un train d'engrenages 112 couplant le moteur au rotor, un module électronique de commande 113 et une pile bâton 114.

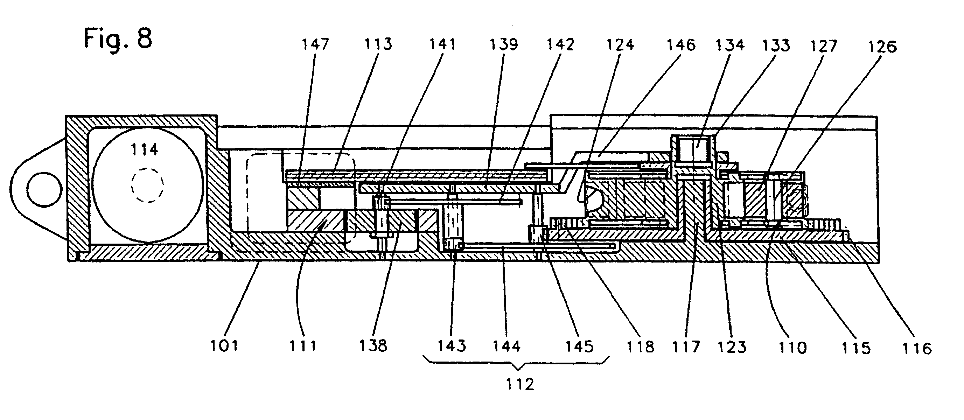

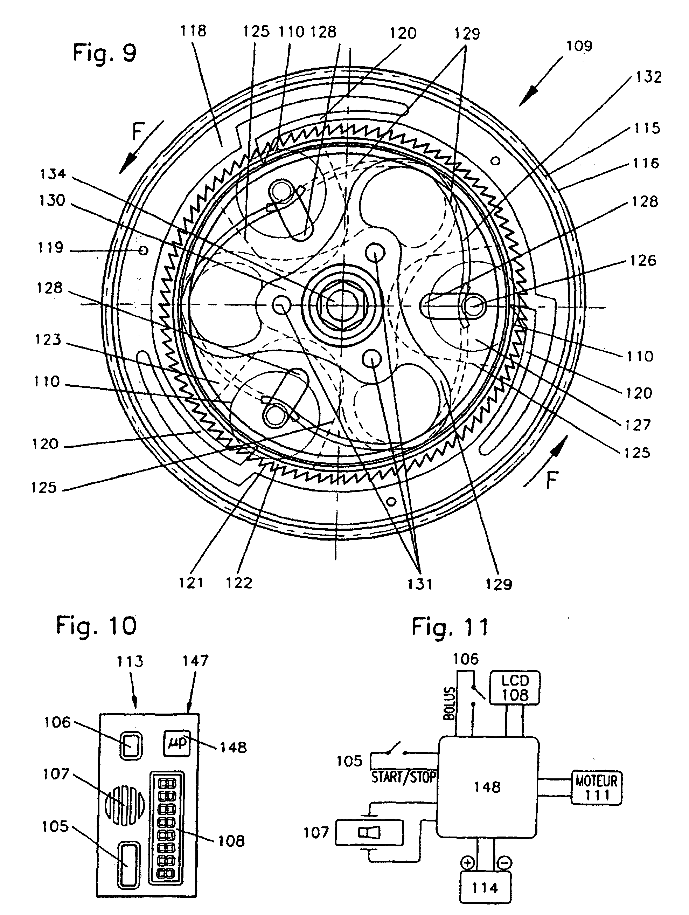

Le rotor 109, représenté en détail sur les figures 8 et 9, comporte une base circulaire 115 munie d'une denture périphérique 116 et montée en libre rotation sur un pivot 117 faisant partie du fond du boitier 101. Une couronne 118, de diamêtre extérieur légèrement inférieur à celui de la base 115, est montée sur cette dernière et fixée à elle par des tenons 119. Cette couronne présente, vers l'intérieur, trois bras souples 120 moulés dans sa masse à 120° l'un de l'autre et formant des cliquets dont l'extrémité coopère avec la périphérie en dents de scie 121 d'une roue 122. Celle-ci constitue la base d'un plateau porte-galets cylindrique 123 monté sur l'axe de la base 115 autour duquel il peut tourner librement.

La paroi périphérique du plateau 123 a un profil incurvé vers l'intérieur 124 de manière à former un canal pour le passage du tuyau souple 202. Cette même paroi est également percée de trois logements 125 en forme de U disposés à 120° l'un de l'autre pour recevoir les trois galets 110 de compression du tuyau.

Chaque galet 110 est formé d'un axe 126 et d'un corps cylindrique 127 monté sur l'axe autour duquel il peut tourner librement. Les deux extrémités de cet axe prennent place dans des ouvertures oblongues 128 ménagées radialement dans les portions du plateau 123 formant les parois planes inférieure et supérieure des logements 125. Les galets sont maintenus en position verticale, c'est à dire avec leur axe parallèle à l'axe du rotor, et soumis à une force radiale dirigée vers l'extérieur grâce à deux ressorts 129 disposés de part et d'autre du plateau 123. Chaque ressort comporte une partie centrale rigide 130, de forme sensiblement triangulaire, fixée au plateau concentriquement à lui par des rivets 131 et trois bras ressorts flexibles recourbés 132 dont les extrémités libres prennent appui sur les extrémités respectives des axes 126 et les poussent vers l'extérieur de manière à ce que les galets 110 exercent sur le tuyau 202 une force sensiblement constante, fixée à 120 grammes dans l'exemple décrit. Les erreurs de positionnement relatif des galets et du tuyau, dues aux inévitables défauts de fabrication, sont ainsi automatiquement compensées, ce qui évite une compression excessive ou insuffisante du tuyau 202.

Le plateau 123 se prolonge axialement vers la haut par une protubérance 133 traversant la partie centrale 130 du ressort supérieur 129. Cette protubérance est percée de l'ouverture de forme 134 déjà citée dans laquelle peut s'introduire, à travers l'orifice circulaire 310 de la cassette, la pointe profilée de manière correspondante d'un outil (non représenté) permettant la rotation rapide du plateau pour effectuer la purge de la pompe.

L'entraînement en rotation du rotor 109 est assuré par le moteur pas à pas 111 qui est du type monophasé bipolaire classique et tourne à la vitesse de 16 tours/seconde à raison de deux pas par tour. Son noyau-bobine et son stator, respectivement désignés par les références 135 et 136, sont fixés sur des pieds formés dans le fond du boîtier 101 par deux vis 137. Son rotor 138 pivote entre le fond du boîtier et un pont 139 fixé par des vis 140 sur des pieds solidaires du fond du boîtier.

Le rotor 138 porte un pignon 141 engrènant avec une roue 142 formant le premier mobile du train d'engrenages 112. Le pignon 143 de cette roue engrène lui-même avec une roue 144 dont le pignon 145 engrène enfin avec la denture périphérique 116 de la base 115 du rotor 109 pour la faire tourner à la vitesse de 0.625 tour par minute. Les roues 142 et 144 pivotent, comme le rotor 138, entre le fond du boîtier et le pont 139. Ce dernier se termine, après un coude, par deux bras 146, non représentés sur la figure 9, dont l'extrémité commune comporte une ouverture circulaire dans laquelle est maintenue et pivote la protubérance 133 du rotor 109.

En fonctionnement, lorsque la base 115 du rotor est entraînée dans le sens de la flèche F par le moteur 111 par l'intermédiaire du train d'engrenages 112, elle fait tourner avec elle la couronne 118 dont elle est solidaire. Les trois cliquets 120 de cette dernière coopèrent alors avec la périphérie en dents de scie 121 de la roue 122 pour la mettre en rotation et, avec elle, le plateau porte-galets 123 dont elle forme la base. Les galets 110 viennent ainsi tour à tour comprimer le tuyau 102 pour pousser le liquide qu'il contient en direction de l'aiguille d'injection 602.

Lorsque, pour réaliser la purge des tubulures, on fait tourner le plateau porte-galets 123 à l'aide d'un outil de forme appropriée introduit dans l'ouverture 134, la base 115 et la couronne 118 deviennent fixes, car freinées par le couple de rétention du moteur 111 et son train d'engrenages 112. Un débrayage s'opère alors automatiquement entre la couronne 118 et la roue 122 du fait que la rotation de celle-ci provoque l'expulsion des trois cliquets 120 des dents de scie 121. Le plateau porte-galets 123 peut ainsi être tourné rapidement sans effet sur le train d'engrenages et le moteur.

Le module électronique de commande 113, représenté sur la figure 10, a pour base une plaquette de circuit imprimé 147 fixée sous la face supérieure du boîtier et portant les boutons 105 et 106, l'avertisseur acoustique 107 et l'affichage LCD 108. La plaquette porte également un circuit microprocesseur 148 intégrant les principales fonctions suivantes : doubleur de tension, base de temps à quartz, mémoire, driver LCD et générateur de son. Ce microprocesseur étant d'un type connu, tel que le EPSON 62L35, il ne sera pas décrit en détail. L'ensemble de ces composants ainsi que la pile 114 et la bobine du moteur 111 sont interconnectés selon le schéma simplifié de la figure 11.

Le microprocesseur 148 est programmé, selon des moyens bien connus de l'homme de métier, pour que l'ensemble fonctionne comme suit.

Lorsque l'interrupteur 105 est actionné pour la première fois, il déclenche le fonctionnement du microprocesseur 148 qui, sous les ordres d'un programme d'administration enregistré dans la mémoire, applique aux bornes du moteur pas à pas 111 des impulsions motrices à la fréquence de 32 Hz provoquant sa rotation à la vitesse de 16 tours/sec. Le rotor porte-galets 109 est alors entraîné à la vitesse de 0.625 tour/min. Le fonctionnement de la pompe est interrompu lorsque l'interrupteur 105 est actionné une deuxième fois.

Pour fixer les idées, avec un rayon de rotation du plateau porte-galets de 10.44 mm et une vitesse de 0.625 tour/min, la solution progresse dans le tuyau à la vitesse de 41 mm/min. En choisissant un tuyau ayant un diamêtre intérieur de 1.47 mm, le débit instantané de la pompe est ainsi de 69.54 mm3/min, soit 100 cm3/jour.

Le microprocesseur 148 permet également, en réponse à l'actionnement du bouton 106, d'échapper au programme d'administration mis en mémoire pour commander l'injection d'une quantité supplémentaire déterminée de solution (bolus). Cette procédure est plus particulièrement destinée aux traitements anti-douleur.

La pompe est également munie de moyens permettant d'actionner l'avertisseur acoustique 107 et/ou l'affichage LCD 108 en cas de mauvais fonctionnement : non respect du programme, arrêt, obstruction de l'aiguille, fin de vie de la pile....Ces moyens ne faisant pas l'objet du présent brevet, ils ne seront pas décrits.

On se référera enfin à la figure 12 qui montre une variante de réalisation du module cassette 300 pour des traitements nécessitant l'injection d'importantes quantités de solution. La cassette peu, certes, être rallongée pour recevoir un réservoir plus grand, mais si le volume de celui-ci dépasse 20 ml, l'encombrement de la pompe risque de la rendre peu pratique. Pour cette raison, le module cassette de la figure 12 ne comporte que le plateau 305 décrit à la figure 3, les mêmes références étant utilisées pour désigner les mêmes éléments. Le module réservoir comprend alors une poche de grand volume 206, qui n'est pas introduite dans de la cassette mais seulement reliée à elle par un tuyau souple 202 plus long identique à celui décrit à la figure 2.

## Revendications

1. Pompe péristaltique miniature pour l'injection de solutions médicamenteuses, comportant un premier (100) et un deuxième (300) module dotés chacun de moyens permettant leur accouplement,
- le premier module (100), dit module pompe, comprenant un rotor (109) équipé d'au moins un galet presseur (110), des moyens d'entraînement (111, 112) dudit rotor et des moyens de commande (113) desdits moyens d'entraînement,
- le deuxième module (300), dit module cassette, comprenant une pièce d'appui munie d'une portion arrondie (308) qui, une fois les deux modules accouplés, est sensiblement concentrique au rotor (109),
**caractérisée en ce que** :
- le module cassette (300) comporte deux canaux (313, 314) ménagés le long de ses côtés pour recevoir un tuyau flexible (202) relié à - et faisant partie indissociable d' - un réservoir de solution médicamenteuse (201) et le positionner automatiquement de manière à ce qu'au moment de l'accouplement des deux modules, il s'applique contre ladite portion arrondie (308) pour y être comprimé par ledit galet (110), et
- lesdits lesdits canaux comportent des portions coudées (315, 316), profilées et dimensionnées pour que des portions (204, 205) de forme correspondante dudit tuyau (202) y prennent place et y soient retenues par enclipsage,
- le tuyau (202) et le réservoir (201) formant ainsi un troisième module de ladite pompe péristaltique (200), dit module réservoir, qui peut être aisément échangé avec un autre après usage.

2. Pompe péristaltique miniature selon la revendication 1, **caractérisée en ce que** le module cassette (300) comprend un plateau (305) s'engageant, comme un tiroir, dans le module pompe (100) et percé d'un logement (307), en forme générale de U, profilé et dimensionné pour entourer le rotor (109) et dont le fond arrondi (308) constitue la pièce d'appui du tuyau.

3. Pompe péristaltique miniature selon la revendication 2, **caractérisée en ce que** le module cassette (300) se fixe dans le module pompe (100) par enclipsage de son plateau (305) dans des coulisses (102) dudit module pompe.

4. Pompe péristaltique miniature selon l'une des revendications précédentes, **caractérisée en ce que** le module cassette (300) comprend, en outre, un logement (301) pour recevoir le réservoir de solution médicamenteuse (201 ).

5. Pompe péristaltique miniature selon l'une des revendications précédentes, **caractérisée en ce que** le rotor (109) comporte un plateau rotatif porte-galet (123) couplé auxdits moyens d'entraînement (111, 112) et possédant un logement (125) ouvert radialement, avec une paroi supérieure et une paroi inférieure entre lesquelles ledit galet (110) est monté rotatif.

6. Pompe péristaltique miniature selon l'une des revendications précédentes, **caractérisée en ce que** le rotor (109) comporte des moyens (129) de compensation automatique des écarts de position entre le galet (110) du module pompe et la pièce d'appui (308) du module cassette.

7. Pompe péristaltique miniature selon la revendication 6, **caractérisée en ce que** le galet (110) comporte un corps (127) sensiblement cylindrique et un axe (126) sur lequel ledit corps est monté rotatif, **en ce que** les extrémités dudit axe prennent place dans des ouvertures oblongues (128) ménagées radialement dans le rotor (109) et **en ce que** lesdits moyens de compensation comportent deux ressorts (129) disposés au niveau des extrémités respectives de l'axe du galet et comprenant chacun une partie centrale (130) concentrique au rotor et un bras ressort (132) recourbé dont une extrémité est solidaire de ladite partie centrale et dont l'autre extrémité prend appui contre l'extrémité correspondante de l'axe du galet pour le pousser radialement vers l'extérieur et permettre ainsi audit galet d'exercer sur le tuyau une force de compression sensiblement constante.

8. Pompe péristaltique miniature selon l'une des revendications précédentes, **caractérisée en ce qu'**elle comporte, en outre, des moyens (133, 134) pour faire tourner ledit rotor (109) par une action externe afin de réaliser la purge du tuyau (202).

9. Pompe péristaltique miniature selon la revendication 8, **caractérisée en ce que** le rotor (109) est réalisé en deux parties concentriques superposées dont la première est couplée auxdits moyens d'entraînement (111, 112) et dont la deuxième porte le galet (110) et **en ce qu'**il comporte, en outre, des moyens d'embrayage-débrayage grâce auxquels:
- en fonctionnement normal, ses deux parties coopèrent pour réaliser sa mise en rotation par ses moyens d'entraînement,
- lorsqu'il est mis en rotation par une action externe, lesdites parties sont automatiquement désaccouplées.

10. Pompe péristaltique miniature selon la revendication 9, **caractérisée en ce que** la première partie du rotor (109) comporte une roue dentée (115) coopérant avec lesdits moyens d'entraînement, **en ce que** sa deuxième partie comporte également une roue dentée (122) formant la base d'un plateau porte-galet (123) et **en ce que** lesdits moyens d'embrayage-débrayage comportent une couronne (118) fixée sur la roue dentée de la première partie concentriquement à elle, entourant la roue dentée de la deuxième partie et présentant, vers l'intérieur, trois bras souples (120) formant des cliquets dont l'extrémité coopère avec la roue dentée de la deuxième partie.

11. Pompe péristaltique miniature selon l'une des revendications 9 et 10, **caractérisée en ce que** les moyens pour faire tourner le rotor par une action externe comportent une protubérance (133) prolongeant l'axe du rotor et percée d'une ouverture de forme (134) destinée à recevoir l'extrémité d'un outil de forme correspondante.

## Claims

1. Miniature peristaltic pump for injecting medicinal solutions, comprising a first (100) and a second (300) module, each provided with means allowing them to be coupled,
- the first module (100), termed the pump module, comprising a rotor (109) equipped with at least one pressing roller (110), means (111, 112) of driving the said rotor and means (113) of controlling the said drive means,
- the second module (300), termed the cassette module, comprising a bearing piece equipped with a rounded portion (308) which, once the two modules have been coupled, is roughly concentric with the rotor (109),
**characterized in that**:
- the cassette module (300) has two ducts (313, 314) formed along its sides to take a flexible hose (202) connected to, and forming an indissociable part of, a reservoir (201) of medicinal solution and for automatically positioning it in such a way that, at the time of coupling of the two modules, it presses against the said rounded portion (308) where it is compressed by the said roller (110), and
- the said ducts have elbowed portions (315, 316) shaped and sized so that correspondingly shaped portions (204, 205) of the said hose (202) fit into them and are held in place by clipping,
- the hose (202) and the reservoir (201) thus forming a third module (200) of the said peristaltic pump, termed the reservoir module, that can easily be exchanged for another when it has been used up.

2. Miniature peristaltic pump according to Claim 1, **characterized in that** the cassette module (300) comprises a plate (305) fitting, in the manner of a slide, in the pump module (100) and pierced with a housing (307), with the overall shape of a U, shaped and sized to surround the rotor (109) and the rounded bottom (308) of which constitutes the bearing piece for the hose.

3. Miniature peristaltic pump according to Claim 2, **characterized in that** the cassette module (300) is fixed into the pump module (100) by clipping its plate (305) into slideways (102) of the said pump module.

4. Miniature peristaltic pump according to one of the preceding claims, **characterized in that** the cassette module (300) further comprises a housing (301) to accommodate the reservoir of medicinal solution (201).

5. Miniature peristaltic pump according to one of the preceding claims, **characterized in that** the rotor (109) comprises a rotary roller-bearing plate (123) coupled to the said drive means (111, 112) and having a radially open housing (125) with a top wall and a bottom wall between which walls the said roller (110) is mounted such that it can rotate.

6. Miniature peristaltic pump according to one of the preceding claims, **characterized in that** the rotor (109) comprises means (129) for automatically compensating for the positional offsets between the roller (110) of the pump module and the bearing piece (308) of the cassette module.

7. Miniature peristaltic pump according to Claim 6, **characterized in that** the roller (110) comprises a roughly cylindrical body (127) and a spindle (126) on which the said body is mounted such that it can rotate, **in that** the ends of the said spindle fit in oblong openings (128) formed radially in the rotor (109), and **in that** the said compensating means comprise two springs (129) arranged at the respective ends of the roller spindle and each comprising a central part (130) concentric with the rotor and a bent spring arm (132) one end of which is secured to the said central part and the other end of which bears against the corresponding end of the roller spindle to push it radially outwards and thus allow the roller to exert a roughly constant compression force on the hose.

8. Miniature peristaltic pump according to one of the preceding claims, **characterized in that** it further comprises means (133, 134) for causing the said rotor (109) to turn through an external action so that the hose (202) can be emptied.

9. Miniature peristaltic pump according to Claim 8, **characterized in that** the rotor (109) is made in two superposed concentric parts, the first of which is coupled to the said drive means (111, 112) and the second of which bears the roller (110), and **in that** it further comprises engagement/disengagement means by virtue of which:
- in normal operation its two parts collaborate to cause it to be rotated by its drive means,
- when it is set in rotation by an external action, the said parts are automatically uncoupled.

10. Miniature peristaltic pump according to Claim 9, **characterized in that** the first part of the rotor (109) comprises a toothed wheel (115) collaborating with the said drive means, **in that** its second part also comprises a toothed wheel (122) forming the base of a roller-bearing plate (123), and **in that** the said engagement/disengagement means comprise an annulus (118) fixed to the toothed wheel of the first part concentrically with respect to the latter, surrounding the toothed wheel of the second part and having, towards the inside, three flexible arms (120) forming pawls, the ends of which collaborate with the toothed wheel of the second part.

11. Miniature peristaltic pump according to one of Claims 9 and 10, **characterized in that** the means for causing the rotor to turn through an external action comprise a protrusion (133) extending the spindle of the rotor and pierced with a shaped opening (134) intended to take the end of a correspondingly shaped tool.

## Patentansprüche

1. Miniaturisierte Peristaltikpumpe zum Injizieren von Arzneimittellösungen mit einem ersten (100) und einem zweiten (300) Modul, die jeweils mit ihre Verbindung gestattenden Mitteln versehen sind,
- wobei das erste Modul (100), das sogenannte Pumpenmodul, einen Rotor (109), der mit mindestens einer Andruckrolle (110) ausgestattet ist, Antriebsmittel (111, 112) des Rotors und Steuermittel (113) der Antriebsmittel enthält,
- wobei das zweite Modul (300), das sogenannte Kassettenmodul, ein Stützteil enthält, das mit einem abgerundeten Teil (308) versehen ist, der nach der Verbindung der beiden Module im Wesentlichen konzentrisch zum Rotor (109) ist,
**dadurch gekennzeichnet, dass**
- das Kassettenmodul (300) zwei Kanäle (313, 314) aufweist, die entlang seinen Seiten zur Aufnahme eines Schlauchs (202), der mit einem Arzneimittellösungsbehälter (201) verbunden ist und damit einen untrennbaren Teil bildet, und zu seiner automatischen Positionierung derart, dass es zu dem Zeitpunkt der Verbindung der beiden Module gegen den abgerundeten Teil (308) gedrückt und dort durch die Rolle (110) zusammengedrückt wird, ausgebildet sind, und
- die Kanäle gekrümmte Teile (315, 316) aufweisen, die so profiliert und dimensioniert sind, dass eine entsprechende Form aufweisende Teile (204, 205) des Schlauchs (202) dort angeordnet und durch Einrasten festgehalten werden,
- der Schlauch (202) und der Behälter (201) somit ein drittes Modul der Peristaltikpumpe (200), das sogenannte Behältermodul, bilden, das nach dem Gebrauch leicht gegen ein anderes ausgetauscht werden kann.

2. Miniaturisierte Peristaltikpumpe nach Anspruch 1, **dadurch gekennzeichnet, dass** das Kassettenmodul (300) ein Tablett (305) aufweist, das wie eine Schublade in das Pumpenmodul (100) eingreift und mit einer allgemein U-förmigen Aufnahme (307) versehen ist, die so profiliert und dimensioniert ist, dass sie den Rotor (109) umgibt, und deren abgerundeter Boden (308) das Teil zum Abstützen des Schlauchs bildet.

3. Miniaturisierte Peristaltikpumpe nach Anspruch 2, **dadurch gekennzeichnet, dass** das Kassettenmodul (300) durch Einrasten seines Tabletts (305) in Gleitführungen (102) des Pumpenmoduls im Pumpenmodul (100) befestigt wird.

4. Miniaturisierte Peristaltikpumpe nach einem der vorhergehenden Ansprüche, **dadurch gekennzeichnet, dass** das Kassettenmodul (300) des Weiteren eine Aufnahme (301) zum Aufnehmen des Arzneimittellösungsbehälters (201) umfasst.

5. Miniaturisierte Peristaltikpumpe nach einem der vorhergehenden Ansprüche, **dadurch gekennzeichnet, dass** der Rotor (109) ein Rollentragdrehtablett (123) aufweist, das mit den Antriebsmitteln (111, 112) verbunden ist und eine radial offene Aufnahme (125) mit einer oberen Wand und einer unteren Wand, zwischen denen die Rolle (110) drehbar angebracht ist, besitzt.

6. Miniaturisierte Peristaltikpumpe nach einem der vorhergehenden Ansprüche, **dadurch gekennzeichnet, dass** der Rotor (109) Mittel (129) zum automatischen Ausgleich von Positionsabweichungen zwischen der Rolle (110) des Pumpenmoduls und dem Stützteil (308) des Kassettenmoduls aufweist.

7. Miniaturisierte Peristaltikpumpe nach Anspruch 6, **dadurch gekennzeichnet, dass** die Rolle (110) einen im Wesentlichen zylindrischen Körper (127) und eine Achse (126) aufweist, auf der der Körper drehbar angebracht ist, dass die Enden der Achse in radial im Rotor (109) ausgebildeten länglichen Öffnungen (128) angeordnet werden, und dass die Ausgleichsmittel zwei Federn (129) umfassen, die an den jeweiligen Enden der Achse der Rolle angeordnet sind und jeweils einen zum Rotor konzentrischen mittleren Teil (130) und einen gebogenen Federarm (132) umfassen, von dem ein Ende fest mit dem mittleren Teil verbunden ist und sich das andere Ende am entsprechenden Ende der Achse der Rolle abstützt, um diese radial nach außen zu schieben und somit zu gestatten, dass die Rolle auf den Schlauch eine im Wesentlichen konstante Druckkraft ausübt.

8. Miniaturisierte Peristaltikpumpe nach einem der vorhergehenden Ansprüche, **dadurch gekennzeichnet, dass** sie des Weiteren Mittel (133, 134) zur Drehung des Rotors (109) durch eine externe Handlung umfasst, um das Spülen des Schlauchs (202) durchzuführen.

9. Miniaturisierte Peristaltikpumpe nach Anspruch 8, **dadurch gekennzeichnet, dass** der Rotor (109) in zwei übereinander liegenden, konzentrischen Teilen ausgebildet ist, von denen der erste mit den Antriebsmitteln (111, 112) verbunden ist und der zweite die Rolle (110) trägt, und dass er des Weiteren Einrück-Ausrück-Mittel umfasst, dank derer:
- bei Normalbetrieb seine beiden Teile zusammenwirken, um seine Drehung durch seine Antriebsmittel zu realisieren,
- wenn er durch eine externe Handlung in Drehung versetzt ist, die Teile automatisch entkuppelt werden.

10. Miniaturisierte Peristaltikpumpe nach Anspruch 9, **dadurch gekennzeichnet, dass** der erste Teil des Rotors (109) ein Zahnrad (115) umfasst, das mit den Antriebsmitteln zusammenwirkt, dass sein zweiter Teil ebenfalls ein Zahnrad (122) umfasst, das die Basis eines Rollentragtabletts (123) bildet, und dass die Einrück-Ausrück-Mittel einen Kranz (118) aufweisen, der am Zahnrad des ersten Teils konzentrisch dazu befestigt ist, wobei er das Zahnrad des zweiten Teils umgibt und nach innen hin drei flexible Arme (120) aufweist, die Sperrklinken bilden, deren Ende mit dem Zahnrad des zweiten Teils zusammenwirkt.

11. Miniaturisierte Peristaltikpumpe nach Anspruch 9 oder 10, **dadurch gekennzeichnet, dass** die Mittel zum Indrehungversetzen des Rotors durch eine externe Handlung einen Vorsprung (133) aufweisen, der die Achse des Rotors verlängert und mit einer Formöffnung (134) versehen ist, die das Ende eines Werkzeugs entsprechender Form aufnehmen soll.
